# NEW EUROPEAN PATENT SPECIFICATION

(11) **EP 1 542 670 B2**
(45) Date of publication and mention of the opposition decision: **05.07.2023**
(45) Mention of the grant of the patent: 15.05.2013
(21) Application number: 03748553.9
(22) Date of filing: 22.09.2003
(51) Int. Cl.: A23L 33/12, A61K 45/06, A61K 31/202, A61P 25/02, A61P 25/28

(54) **COMPOSITION CONTAINING ARACHIDONIC ACID ALONE OR IN COMBINATION WITH DOCOSAHEXAENOIC ACID FOR ENHANCING COGNITIVE ABILITIES IN ADULTS**
ZUSAMMENSETZUNG, ENHALTEND ARACHIDONSAEURE ALLEIN ODER IN KOMBINATION MIT DOCOSAHEXAENSAEURE ZUR VERBESSERUNG DER KOGNITIVEN FûHIGKEITEN BEI ERWACHSENEN.
COMPOSITION CONTENANT DE L'ACIDE ARACHIDONIQUE SEUL OU EN COMBINAISON AVEC DE L'ACIDE DOCOSAHEXANOIQUE UTILE POUR AMELIORER LES CAPACITES COGNITIVES CHEZ L'ADULTE

(30) Priority: 24.09.2002 JP 2002277305
(43) Date of publication of application: 22.06.2005
(73) Proprietor: Suntory Holdings Limited, Kita-ku, Osaka-shi, Osaka 530-8203 (JP)
(72) Inventor: AKIMOTO, Kengo, Kawasaki-shi Kanagawa, 211-0036 (JP); KOGA, Yoshihiko, Setagaya-ku, Tokyo 158-0093 (JP)
(74) Representative: Mewburn Ellis LLP
(86) International application number: PCT/JP2003/012107
(87) International publication number: WO 2004/028529

(56) References cited:
- EP-A- 1 239 022
- WO-A-96/10922
- WO-A-96/21037
- US-A- 4 668 704
- US-A- 6 034 130
- US-A- 6 080 787
- KOLETZKO B ET AL: "Polyunsaturated fatty acids in human milk and their role in early infant development." JOURNAL OF MAMMARY GLAND BIOLOGY AND NEOPLASIA. UNITED STATES JUL 1999, vol. 4, no. 3, July 1999 (1999-07), pages 269-284, XP009024376 ISSN: 1083-3021
- CARLSON S E: "Docosahexaenoic acid and arachidonic acid in infant development." SEMINARS IN NEONATOLOGY: SN. ENGLAND OCT 2001, vol. 6, no. 5, October 2001 (2001-10), pages 437-449, XP008024374 ISSN: 1084-2756
- AUESTAD NANCY ET AL: "Visual, cognitive, and language assessments at 39 months: a follow-up study of children fed formulas containing long-chain polyunsaturated fatty acids to 1 year of age." PEDIATRICS. UNITED STATES SEP 2003, vol. 112, no. 3 Pt 1, September 2003 (2003-09), pages e177-e183, XP009024347 ISSN: 1098-4275
- WILLATTS P ET AL: "Effect of long-chain polyunsaturated fatty acids in infant formula on problem solving at 10 months of age" LANCET, XX, XX, vol. 352, no. 9129, 29 August 1998 (1998-08-29), pages 688-691, XP004265590 ISSN: 0140-6736
- LUCAS A A ET AL: "Efficacy and safety of long-chain polyunsaturated fatty acid supplementation of infant-formula milk: a randomised trial" LANCET, XX, XX, vol. 354, no. 9194, 4 December 1999 (1999-12-04), pages 1948-1954, XP004262925 ISSN: 0140-6736
- MCGAHON B M ET AL: "Age-related changes in synaptic function: Analysis of the effect of dietary supplementation with [omega]-3 fatty acids", NEUROSCIENCE, NEW YORK, NY, US LNKD- DOI:10.1016/S0306-4522(99)00219-5, vol. 94, no. 1, 1 September 1999 (1999-09-01), pages 305-314, XP009134730, ISSN: 0306-4522 [retrieved on 1999-09-13]
- M. Beukers.et .al.,. Pharmaceutisch Weekblad Scientific edition.13(1), 1991, 7-12
- P Willatts et al, The Lancet 352, 1998, 688-91

## Description

### FIELD OF THE INVENTION

The present invention provides use of foods and beverages with effects of improvement or enhancement of normal responses of cognitive abilities of a healthy adult containing, as active ingredients, arachidonic acid and/or compounds with arachidonic acid as a constituent fatty acid.

More specifically, the invention provides uses of foods and beverages with effects of improvement or enhancement of normal responses of cognitive abilities of a healthy adult e.g. in awareness level and/or discriminatory ability with respect to events selected from the group consisting of auditory stimuli, visual stimuli, gustatory stimuli, olfactory stimuli and somatosensory stimuli, which contain, as active ingredients, one or more species selected from the group consisting of arachidonic acid, arachidonic acid alcohol esters and triglycerides, phospholipids or glycolipids containing arachidonic acid as part or all of the constituent fatty acid.

### BACKGROUND ART

Cognition is the process of selecting information from the external environment and clearly identifying it, and more specifically, of transmitting external stimuli such as visual, auditory, olfactory, gustatory and somatosensory stimuli to the brain through sensory organs and processing and precisely identifying them by coordinated functioning of multiple regions of the brain. Cognitive abilities consist of the two factors of information processing speed (speed of transforming external stimuli) and the amount of information processing resources (the level of resource allocation for information processing on external stimuli).

The effects of improvement or enhancement of normal responses of cognitive abilities according to the present disclosure may relate to normal functions such as attention, memory, perception, language, calculation and the like, while being distinct from improvement of impaired function.

Various therapeutic agents have been researched and developed in relation to impaired cognitive function. Unfortunately, however, no effective compounds have yet been found that prevent decline of, improve or enhance normal responses of cognitive abilities of a healthy person. That is, although methods are known for determining cognitive function impairment and evaluating improving effects (see, for example, Nishimura, T., Takeda, M., "Diagnostic imaging of Alzheimer's dementia", pp.27-36, Medical View Publications (2/10/2001)) are known, such methods do not allow evaluation of decline prevention, improvement or enhancement of normal responses. A need has therefore existed for an effective means of objectively evaluating normal responses of cognitive abilities of a healthy person in order to objectively develop and research drug agents for decline prevention, improvement or enhancement of normal responses of cognitive abilities of a healthy person.

Attention has recently become focused on event related potentials of brain as a physiological index to serve as an objective evaluation of cognitive ability. It is possible to record the electrical activity exhibited by neurons through electrodes attached on the human scalp. Background brain waves are generally known as continuous electrical activity which varies depending on the arousal level. A transient minute action potential is also present which varies in relation to a given specifiable event. This action potential is currently referred to as the "event related potentialss of brain", and is interpreted as the potential induced by internal or external stimuli. The event related potentialss of brain, a minute potential variation of about 0.1 µV to several tens of µV, is composed of a series of positive waves and negative waves. The positive wave appearing near 300 msec for the stimulus event among this series of potential variation is associated with cognitive function and is termed P300. The time to appearance of P300 for a stimulus event is called the P300 latency and represents information processing speed (speed of transforming external stimuli), while the height of the peak from the baseline is called the P300 amplitude and represents the amount of information processing resources (the level of resource allocation for information processing on external stimuli) (see, for example, Mitamura, S., "Current Therapy", Vol.18, No.4, pp.618-621, Life Medicom (2/10/2001)). It has thus become possible to objectively measure human cognitive response by measuring the event related potentialss of brain.

External stimuli are transmitted through sensory organs to the brain, and the brain is the tissue like adipose mass, about 1/3 of the white matter and 1/4 of the gray matter consists of phospholipids. Because most of the polyunsaturated fatty acids of the phospholipids constituting the cell membranes of the brain are arachidonic acid and docosahexaenoic acid, it has been suggested that these polyunsaturated fatty acids may play some role in enhancing learning and memory abilities and preventing or improving senile dementia. Arachidonic acid and docosahexaenoic acid cannot be synthesized de *novo* in animals and therefore must be ingested from food either directly or indirectly (as linoleic acid precursor of arachidonic acid or α-linoleic acid precursor of docosahexaenoic acid). Attention has thus been focused on enhancing effects on learning and memory and preventive and improving effects on senile dementia by externally supplying arachidonic acid and docosahexaenoic acid. Docosahexaenoic acid is found abundantly in fish oil sources and a great deal of research has been conducted on its effects of brain function improvement, while inventions have been disclosed in regard to its use in learning ability reinforcers, memory reinforcers, dementia preventing agents, dementia treatment agents, anti-dementia agents or functional foods with brain function-improving effects (see, for example, Japanese Unexamined Patent Publication No. 7-82146, Japanese Unexamined Patent Publication No. 5-117147 and Japanese Unexamined Patent Publication No. 2-49723). Furthermore, arachidonic acid and/or compounds containing arachidonic acid as a constituent fatty acid have recently been shown by the present inventors, to improve age-related decline in learning ability, based on results of administering arachidonic acid and/or a compound comprising arachidonic acid as a constituent fatty acid to aged animals subjected to a Morris water maze test, as described in Japanese Unexamined Patent Publication No. 2003-48831, which was published on February 21, 2003, entitled "Composition Having Effects Of Preventing Or Ameliorating Conditions Or Diseases Caused By Brain Hypofunction". However, this particular invention is directed toward decline in brain function and discloses nothing regarding effects on normal responses of cognitive abilities of a healthy person.

Some attempts have been made to determine the improving effects of several compounds on cognitive response based on event related potentialss of brain. Miyanaga, K. examined the pharmacological action of DHA on brain function, measuring event related potentials of brain before and after orally administering capsules containing 2400 mg DHA to 26 healthy subjects, and discovered that the P300 latency was significantly shortened and the P300 amplitude was significantly augmented (see Miyanaga, K., "Shoku no Kagaku" [Food Science] pp.84-96, Korin Books (1999)). However, due to a lack of comparative results with placebo samples, no correlation could be established between blood DHA levels and P300 results. In a prolonged administration test with daily administration of capsules containing 900 mg DHA to 97 healthy geriatric subjects for 6 months, no change in P300 was observed, thus leaving uncertainty about the efficacy of DHA.

Therefore, while docosahexaenoic acid is considered to exhibit an improving effect on learning ability, the fact that no effective results were found in terms of the event related potentials of brain as an index of cognitive ability means that it remains unclear whether or not arachidonic acid and/or compounds with arachidonic acid as a constituent fatty acid are effective for decline prevention, improvement and enhancement of normal responses of cognitive abilities of a healthy person.

McGahon et al. (Neuroscience, Vol. 94, No. 1, pp. 305-314, 1999) describes dietary supplementation with ω-3 fatty acids, DHA in particular, in rats. It notes some improvement in age-related impairment of hippocampal long-term potentiation in rats fed a diet supplemented by ω-3 fatty acids, DHA in particular, in tuna oil.

### DISCLOSURE OF THE INVENTION

### Problems to be Solved by the Invention

Consequently, it has been ardently desired to develop compounds which prevent decline of, improve or enhance normal responses of information processing speed (speed of transforming external stimuli) and the amount of information processing resources (the level of resource allocation for information processing on external stimuli), as the two factors of cognitive abilities, and which are highly suitable for pharmaceuticals as well as food products, with minimal side effects.

### Means for Solving the Problems

The present invention therefore provides use according to claim 1, e.g. of foods and beverages.

More specifically, the disclosure provides use of compositions, foods and beverages with effects of improvement or enhancement of normal responses of cognitive abilities of a healthy adult awareness level and/or discriminatory ability with respect to events (auditory stimuli, visual stimuli, gustatory stimuli, olfactory stimuli, somatosensory stimuli), which containes as active ingredients one or more species selected from the group consisting of arachidonic acid, arachidonic acid alcohol esters and triglycerides, phospholipids or glycolipids comprising arachidonic acid as part or all of the constituent fatty acid.

As a result of diligent research for the purpose of elucidating the effects of improvement or enhancement of normal responses of cognitive abilities of a healthy person by arachidonic acid and/or compounds comprising arachidonic acid as a constituent fatty acid, the present inventors surprisingly discovered an effect of arachidonic acid and/or compounds comprising arachidonic acid as a constituent fatty acid, based on evaluation using the event related potentials of brain as an index.

The present inventors also succeeded in achieving microbial industrial production of triglycerides containing at least 10 wt% arachidonic acid, and elucidated the effects of those triglycerides in effects tests.

The present inventors further succeeded in achieving enzymatic production of oils and fats containing triglycerides with medium-chain fatty acids bound at the 1,3-positions and arachidonic acid bound at the 2-position, and elucidated the effects of those triglycerides in effects tests.

The present disclosure therefore provides uses of compositions, foods and beverages with effects of improvement or enhancement of normal responses of cognitive abilities of a healthy person, containing as active ingredients arachidonic acid and/or compounds with arachidonic acid as a constituent fatty acid. More specifically, the disclosure provides foods and beverages with effects of improvement or enhancement of normal responses of cognitive abilities of a healthy person, awareness level and/or discriminatory ability with respect to events (auditory stimuli, visual stimuli, gustatory stimuli, olfactory stimuli, somatosensory stimuli), which contains as active ingredients one or more species selected from the group consisting of arachidonic acid, arachidonic acid alcohol esters and triglycerides, phospholipids or glycolipids comprising arachidonic acid as part or all of the constituent fatty acid.

According to the disclosure it is possible to provide foods and beverages having effects of improvement or enhancement of normal responses of cognitive abilities of a healthy person which contains as active ingredients arachidonic acid and/or compounds with arachidonic acid as a constituent fatty acid, thus the invention is particularly useful in modern society.

### BRIEF DESCRIPTION OF THE DRAWINGS

Fig. 1 is a bar graph showing changes in arachidonic acid levels in serum phospholipids before and after ingestion of arachidonic acid-containing edible oil capsules and olive oil-containing capsules (placebo capsules).
Fig. 2 is a line graph showing changes in P300 latency before and after ingestion of arachidonic acid-containing edible oil capsules and olive oil-containing capsules (placebo capsules).
Fig. 3 is a line graph showing changes in P300 amplitude before and after ingestion of arachidonic acid-containing edible oil capsules and olive oil-containing capsules (placebo capsules).
Fig. 4 is a pair of dot graphs showing correlation between P300 (P300 latency and amplitude) and arachidonic acid levels in serum phospholipids.

### DETAILED DESCRIPTION OF THE INVENTION

The present invention relates to use of compositions, foods and beverages with effects of improvement or enhancement of normal responses of cognitive abilities of a healthy adult , comprising as active ingredients arachidonic acid and/or compounds with arachidonic acid as a constituent fatty acid.

The compositions used herein have effects of improvement or enhancement of normal responses of cognitive abilities of a healthy adult , or stated differently, they have effects of improvement or enhancement of normal responses of awareness level of a healthy person and have effects of improvement or enhancement of normal responses of discriminatory ability of a healthy person with respect to events (auditory stimuli, visual stimuli, gustatory stimuli, olfactory stimuli, somatosensory stimuli), and are able to exhibit their effects in foods or beverages, pharmaceuticals, quasi drugs and the like. The effects of improvement or enhancement of normal responses of cognitive abilities of a healthy adult are also effective as foods or beverages and quasi drugs.

The active ingredients may be, instead of free arachidonic acid, any compounds having arachidonic acid as a constituent fatty acid. Compounds having arachidonic acid as a constituent fatty acid include arachidonic acid salts such as calcium and sodium salts, or arachidonic acid alcohol esters such as arachidonic methyl ester, arachidonic acid ethyl ester and the like. Triglycerides, diglycerides, monoglycerides, phospholipids or glycolipids containing arachidonic acid as part or all of the constituent fatty acid may also be used.

For application to a food product, the arachidonic acid is preferably in the form of triglycerides or phospholipids, and especially triglycerides. While virtually no natural sources of arachidonic acid-containing triglycerides (or triglycerides including triglycerides with arachidonic acid as part or all of the constituent fatty acid) are known, the present inventors have succeeded in enabling industrial use of triglycerides containing arachidonic acid as a constituent fatty acid, and have been the first to elucidate the effect of the active ingredient in humans by prolonged administration to humans and analysis of event related potentials of brain allowing objective evaluation of cognitive abilities, and to demonstrate that it has an effect of improvement or enhancement of normal responses of cognitive abilities of a healthy person.

According to the present invention, therefore, there may be used triglycerides which include triglycerides wherein part or all of the constituent fatty acids are arachidonic acid as the active ingredient (arachidonic acid-containing triglycerides). For application to food products, the arachidonic acid-containing triglycerides are preferably in the form oils or fats (triglycerides) wherein the proportion of arachidonic acid is at least 20 wt% (W/W), preferably at least 30 wt% and more preferably at least 40 wt% of the total fatty acids of the triglycerides. According to the invention, therefore, there may be used any arachidonic acid-containing oils or fats (triglycerides) obtained by culturing microbes capable of producing the same.

As examples of microbes capable of producing arachidonic acid-containing oils or fats (triglycerides) there may be mentioned microbes belonging to the genus *Mortierella, Conidiobolus, Pythium, Phytophthora, Penicillium, Cladosporium, Mucor, Fusarium, Aspergillus, Rhodotorula, Entomophthora, Echinosporangium* or *Saprolegnia.* As microbes belonging to the genus *Mortierella* subgenus *Mortierella* there may be mentioned *Mortierella elongata, Mortierella exigua, Mortierella hygrophila, Mortierella alpina,* and the like. Specifically, there may be mentioned strains such as *Mortierella elongata* IFO8570, *Mortierella exigua* IFO8571, *Mortierella hygrophila* IFO5941, *Mortierella alpina* IFO8568, ATCC16266, ATCC32221, ATCC42430, CBS219.35, CBS224.37, CBS250.53, CBS343.66, CBS527.72, CBS529.72, CBS608.70, CBS754.68, etc.

These strains are all available without restriction from the Institute for Fermentation, Osaka (IFO), the American Type Culture Collection (ATCC) and the Centralbureau voor Schimmelcultures (CBS). In addition, there may be used strain *Mortierella elongata* SAM0219 (FERM P-8703) (FERM BP-1239) separated from soil by the research group of the present inventors.

For culturing of the strains used for the invention, the spores, hypha or cells of a seed culture solution obtained by preculturing are inoculated to liquid or solid medium for main culturing. In the case of a liquid medium, the carbon source used may be any commonly used one such as glucose, fructose, xylose, saccharose, maltose, solubilized starch, molasses, glycerol or mannitol, although there is no limitation to these. As nitrogen sources there may be used organic nitrogen sources, including natural nitrogen sources such as peptone, yeast extract, malt extract, meat extract, casamino acid, corn steep liquor, soybean protein, defatted soybean, cottonseed meal or the like, as well as urea, and inorganic nitrogen sources such as sodium nitrate, ammonium nitrate and ammonium sulfate. If necessary, trace nutrient sources including inorganic salts such as phosphoric acid salts, magnesium sulfate, iron sulfate and copper sulfate or vitamins may also be used. These medium components are not particularly restricted so long as they are at concentrations which do not inhibit growth of the microbes. In practice, the carbon source will generally be added in a total amount of 0.1-40 wt% (W/V) and preferably 1-25 wt% (W/V). The initial nitrogen source addition may be at 0.1-10 wt% (W/V) and preferably 0.1-6 wt% (W/V), with further addition of the nitrogen source during culturing if desired.

Oils or fats (triglycerides) containing at least 45 wt% (W/W) arachidonic acid may be prepared as the active ingredient for the invention by controlling the carbon source concentration in the medium. The culturing produces cells in the growth phase up to day 2-4 of culturing, and cells in the oil/fat accumulating phase after day 2-4 of culturing. The initial carbon source concentration is 1-8 wt% and preferably 1-4 wt%, and the carbon source is successively added only during the initial periods of the cell growth phase and oil/fat accumulating phase, up to a total successive carbon source addition of 2-20 wt% and preferably 5-15 wt%. The successive addition of the carbon source during the initial periods of the cell growth phase and oil/fat accumulating phase is based on the initial nitrogen source concentration, for a carbon source concentration of zero in the medium from the 7th day of culturing, preferably from the 6th day of culturing and more preferably from the 4th day of culturing, to obtain oils or fats (triglycerides) comprising at least 45 wt% arachidonic acid, as an active ingredient.

The culturing temperature for the arachidonic acid-producing microbe will differ depending on the microbe, but may be 5-40°C and preferably 20-30°C, or the cells may be cultured at 20-30°C for growth and the culturing continued at 5-20°C to produce unsaturated fatty acids. Such temperature management can also be used to increase the proportion of polyunsaturated fatty acids among the produced fatty acids. The pH of the medium may be 4-10 and preferably 5-9, and the culturing method may be submerged culturing, shake culturing, or stationary culturing. The culturing will usually be conducted for 2-30 days, preferably for 5-20 days and more preferably for 5-15 days.

As a means for increasing the proportion of arachidonic acid in the arachidonic acid-containing oils or fats (triglycerides) other than controlling the carbon source concentration in the medium, the arachidonic acid-containing oils or fats may be selectively hydrolyzed to obtain arachidonic acid-rich oils or fats. Lipases used for selective hydrolysis are not specific to triglyceride positions and, as the hydrolytic activity is lower in proportion to the number of double bonds, fatty acid ester bonds other than those of polyunsaturated fatty acids are hydrolyzed. Also, transesterification occurs between the produced PUFA triglycerides, resulting in triglycerides with increased polyunsaturated fatty acids (see, for example, Enhancement of Arachidonic Acid: Selective Hydrolysis of a Single-Cell Oil from Mortierella with Candida cylindracea lipase", J. Am. Oil Chem. Soc., 72, pp. 1323-1327, AOCS Press (1998)). Thus, arachidonic acid-rich oils or fats (triglycerides) obtained by selective hydrolysis of arachidonic acid-containing oils or fats may be used as active ingredients. A higher proportion of arachidonic acid is preferred with respect to the total fatty acids in arachidonic acid-containing oils or fats (triglycerides) in order to minimize the effects of the other fatty acids, but there is no restriction to a high proportion and, in practice, the absolute amount of arachidonic acid can pose a problem for application to food products, although oils or fats (triglycerides) containing arachidonic acid at 10 wt% or more may be used in practice.

As triglycerides comprising arachidonic acid as part or all of the constituent fatty acid there may be used triglycerides with medium-chain fatty acids bound at the 1,3-positions and arachidonic acid bound at the 2-position. There may also be used oils or fats (triglycerides) comprising at least 5 mole percent, preferably at least 10 mole percent, more preferably at least 20 mole percent and most preferably at least 30 mole percent of triglycerides with medium-chain fatty acids bound at the 1,3-positions and arachidonic acid bound at the 2-position. The medium-chain fatty acids bound at the 1,3-positions of the triglycerides may be selected from among fatty acids of 6 to 12 carbons. As examples of fatty acids of 6 to 12 carbons there may be mentioned caprylic acid and capric acid, with 1,3-capryloyl-2-arachidonoyl-glycerol (hereinafter referred to as "8A8") being preferred.

Triglycerides with medium-chain fatty acids bound at the 1,3-positions and arachidonic acid bound at the 2-position are most suitable as oils or fats (triglycerides) for elderly persons. Ingested oils or fats (triglycerides) are usually hydrolyzed by pancreatic lipases upon entering the small intestine, and since pancreatic lipases are 1,3-position specific type, the 1,3-positions of the triglycerides are cleaved to produce two free fatty acid molecules while simultaneously producing one 2-monoacylglyceride (MG) molecule. Because 2-MG has very high bile acid solubility and is highly absorbable, 2-position fatty acids are generally considered to be better absorbed. Also, 2-MG exhibits surfactant action when dissolved in bile acids, and thus functions to increase the absorption of the free fatty acids. The free fatty acids and 2-MG then form bile acid complex micelles with cholesterol or phospholipids and are taken up by intestinal epithelial cells, resulting in resynthesis of triacylglycerols and finally release into the lymph as chylomicrons. However, the fatty acid properties of the pancreatic lipases are highest for saturated fatty acids, while arachidonic acid is cleaved less efficiently. An additional problem is that pancreatic lipase activity is lower in the elderly, such that the ideal oils or fats (triglycerides) are triglycerides with medium-chain fatty acids bound at the 1,3-positions and arachidonic acid bound at the 2-position.

One specific production process for triglycerides with medium-chain fatty acids bound at the 1,3-positions and arachidonic acid bound at the 2-position is a production process whereby lipase is allowed to act only on 1,3-ester bonds of triglycerides in the presence of arachidonic- containing oils or fats (triglycerides) and medium-chain fatty acids.

The oil or fat (triglycerides) used as the starting material consists of triglycerides with arachidonic acid as a constituent fatty acid, and when the proportion of arachidonic acid is high with respect to the total fatty acids of the triglycerides, a temperature of 30-50°C and preferably 40-50°C is employed which is higher than the ordinary enzyme reaction temperature of 20-30°C, in order to prevent reduction in reaction yield due to increase in unreacted oil or fat (the triglyceride starting material and the triglycerides wherein only one of the 1,3-position fatty acids is a medium-chain fatty acid).

As lipases which act specifically on the 1,3-position ester bonds of triglycerides there may be mentioned those produced by microbes belonging to the genus *Rhizopus, Rhizomucor, Aspergillus* or the like, as well as pig pancreatic lipases. Such lipases may be commercially available ones. Examples thereof include *Rhizopus delemar* lipase (Talipase, product of Tanabe Seiyaku), *Rhizomucor miehei* lipase (Ribozyme IM by Novo Nordisk Co., Ltd.) and *Aspergillus niger* lipase (Lipase A, product of Amano Enzyme Co., Ltd.), with no particular limitation to these enzymes, as any 1,3-position specific type lipases may be used.

The form in which the lipase is used is preferably as an immobilized lipase on an immobilizing support, in order to confer heat stability to the enzyme as the reaction temperature is 30°C or higher and preferably 40°C or higher for increased reaction efficiency. Highly porous resins are used as immobilizing supports, and there may be mentioned ion-exchange resin supports with pores of at least about 100 angstroms, such as Dowex MARATHON WBA (trademark of Dow Chemical).

A 0.5 to 20 times weight of aqueous solution of the 1,3-position specific type lipase with respect to the immobilizing support is suspended therein, and a 2 to 5 times amount of cold acetone (for example, -80°C) with respect to the suspension is slowly added while stirring to form a precipitate. The precipitate is dried under reduced pressure to prepare the immobilized enzyme. As a simpler method, a 0.05 to 0.4 times amount of the 1,3-position specific type lipase with respect to the immobilizing support is dissolved in a minimal amount of water, and then the solution is mixed with the immobilizing support while stirring and the mixture is dried under reduced pressure to prepare the immobilized enzyme. This procedure results in immobilization of about 90% of the lipase on the support but with absolutely no exhibited transesterification activity, and therefore the immobilized enzyme may be activated and provided for production most efficiently by pretreatment in the substrate (raw material oil or fat and medium-chain fatty acids) containing 1 to 10 wt% (W/V) water, and preferably in the substrate containing 1 to 3 wt% water.

The amount of water added to the reaction system is extremely important depending on the type of enzyme, since transesterification will not proceed in the absence of water, while an excess of water causes hydrolysis and a reduced glyceride yield (with diglycerides and monoglycerides being produced upon hydrolysis). In such cases, however, the immobilized enzyme activated by pretreatment may be used to lessen the importance of the amount of water added to the reaction system, allowing transesterification reaction to occur efficiently even in a system containing no water. Moreover, the type of enzyme agent may also be selected to allow omission of pretreatment.

Thus, by using a heat-resistant immobilized enzyme and increasing the enzyme reaction temperature, it is possible to efficiently produce triglycerides with medium-chain fatty acids bound at the 1,3-positions and arachidonic acid bound at the 2-position without reduction in reaction efficiency, even in the case of arachidonic acid-containing oils or fats (triglycerides) with low reactivity for 1,3-position specific type lipases.

The process for production of foods and beverages with effects of improvement or enhancement of normal responses of cognitive abilities of a healthy person employs arachidonic acid and/or a compound with arachidonic acid as a constituent fatty acid, either alone or in admixture with a food or beverage material containing essentially no, or only a trace amount of, arachidonic acid. Here, "trace amount" means that arachidonic acid is present in the food or beverage material but in an amount which does not reach the daily intake of arachidonic acid as described hereunder, if the food composition is ingested by an individual.

Oils and fats (triglycerides) containing arachidonic acid as part or all of the constituent fatty acid have unlimited possibilities for use as raw materials and additives in foods, beverages, pharmaceuticals and quasi drugs. Furthermore, there are no limitations on the purpose or amounts of their use.

As examples of food compositions there may be mentioned common food products as well as functional foods, nutritional supplements, maternal foods or geriatric foods. As examples of food products containing oils or fats there may be mentioned natural foods which originally contain oils and fats, such as meat, fish and nuts, food products with oils and fats added during preparation, such as soups, food products using oils or fats as heating media, such as donuts, oil or fat food products such as butter, processed food products with oils or fats added during processing, such as cookies, or food products sprayed or coated with oils or fats during final processing, such as hard biscuits. They may also be added to fermented foods or beverages which contain no oils or fats. They may also be in the form of functional food products, pharmaceuticals or quasi drugs, and for example, in the form of enteral nutrients, powders, granules, lozenges, oral solutions, suspensions, emulsions, syrups or the like.

The composition may also contain, in addition to the active ingredient, various carriers and additives commonly used in foods and beverages, pharmaceuticals or quasi drugs. In particular it preferably contains antioxidants in order to prevent oxidation of the active ingredient. As antioxidants there may be mentioned natural antioxidants such as tocopherols, flavone derivatives, phyllodulcin, kojic acid, gallic acid derivatives, catechins, fukiic acid, gossypol, pyrazine derivatives, sesamol, guaiaol, guaiac acid, p-coumaric acid, nordihydroguaiatic acid, sterols, terpenes, nucleic acid bases, carotenoids, lignans and the like, and synthetic antioxidants such as ascorbic palmitic ester, ascorbic stearic ester, butylhydroxyanisole (BHA), butylhydroxytoluene (BHT), mono-t-butylhydroquinone (TBHQ) and 4-hydroxymethyl-2,6-di-t-butylphenol (HMBP). As tocopherols there may be mentioned α-tocopherol, β-tocopherol, γ-tocopherol, δ-tocopherol, ε-tocopherol, ξ-tocopherol, η-tocopherol and tocopherol esters (acetic acid tocopherol, etc.). As examples of carotenoids there may be mentioned β-carotene, cantaxanthin, astaxanthin and the like.

As supports to be used in the composition in addition to the active ingredient, there may be mentioned various types of carrier supports, extenders, diluents, fillers, dispersing agents, excipients, binder solvents (for example, water, ethanol or vegetable oils), solubilizing aids, buffers, dissolution accelerators, gelling agents, suspending agents, wheat flour, rice flour, starch, corn starch, polysaccharides, milk protein, collagen, rice oil, lecithin and the like. As examples of additives there may be mentioned vitamins, sweeteners, organic acids, coloring agents, flavorings, dehumidifying agents, fibers, electrolytes, minerals, nutrients, antioxidants, preservatives, aromatic agents, humidifiers, natural edible extracts, vegetable extracts and the like, although there is no limitation to these.

The principal medicinal component of the arachidonic acid or the compound with arachidonic acid as a constituent fatty acid is arachidonic acid. The daily intake of arachidonic acid from food is reported to be 0.14 g in the Kanto region, Japan and 0.19-0.20 g in the Kansai region, Japan (see "Shishitsu Eiyogaku" [Lipid Nutrition] 4, ed. by the Japan Society for Lipid Nutrition Editing Committee, pp.73-82(1995) ISN 1343-4594 CODEN: SHEIFG), and therefore arachidonic acid must be ingested in a corresponding amount or greater. Thus, the daily intake of arachidonic acid or a compound with arachidonic acid as a constituent fatty acid for an adult (60 kg body weight, for example) is 0.001-20 g, preferably 0.01-10 g, more preferably 0.05-5 g and most preferably 0.1-2 g, in terms of arachidonic acid.

When the active ingredient is actually used in a food or beverage product, the absolute amount of arachidonic acid in the food product is also important. However, since the absolute amount in the food or beverage also varies depending on the amount of ingestion of the food or beverage, the food product preferably contains the triglycerides including triglycerides with arachidonic acid as part or all of the constituent fatty acid, in an amount of at least 0.0003 wt%, preferably at least 0.003 wt% and more preferably at least 0.03 wt% in terms of arachidonic acid. When triglycerides with medium-chain fatty acids bound at the 1,3-positions and arachidonic acid bound at the 2-position are added to a food or beverage, the amount of the triglycerides with medium-chain fatty acids bound at the 1,3-positions and arachidonic acid bound at the 2-position is at least 0.001 wt%, preferably at least 0.01 wt% and more preferably at least 0.1 wt%.

The major fatty acids of brain phospholipid membranes are arachidonic acid and docosahexaenoic acid, and therefore the composition is preferably a combination of arachidonic acid and docosahexaenoic acid in consideration of maintaining balance. Generally speaking, arachidonic acid (n-6 unsaturated fatty acid) and docosahexaenoic acid (n-3 unsaturated fatty acid) are biosynthesized by the same enzyme from linoleicacid and α-linolenic acid, respectively. When arachidonic acid is administered alone, therefore, biosynthesis of docosahexaenoic acid is inhibited. Conversely, when docosahexaenoic acid is administered alone, biosynthesis of arachidonic acid is inhibited. In order to avoid such unbalances, it is preferred for arachidonic acid and docosahexaenoic acid to be consumed in combination. Furthermore, as the proportion of eicosapentaenoic acid is very low in brain phospholipid membranes, the combination preferably contains virtually no eicosapentaenoic acid. It therefore preferably contains no eicosapentaenoic acid, or only up to 1%. The composition more preferably contains virtually no eicosapentaenoic acid with the arachidonic acid and docosahexaenoic acid. The combination of arachidonic acid and docosahexaenoic acid also preferably has an arachidonic acid/docosahexaenoic acid ratio (by weight) in the range of 0.1-15 and preferably in the range of 0.25-10. Most preferred are foods and beverages containing eicosapentaenoic acid at no more than 1/5 (by weight) of the arachidonic acid.

### EXAMPLES

The present invention will now be explained in greater detail through the following examples.

### Example 1

### Production process for triglycerides with arachidonic acid as a constituent fatty acid

*Mortierella alpina* was used as an arachidonic acid-producing strain. A 6 kL portion of medium containing 1.8% glucose, 3.1% defatted soybean flour, 0.1% soybean oil, 0.3% KH₂PO₄, 0.1% Na₂SO₄, 0.05% CaCl₂·2H₂O and 0.05% MgCl₂·6H₂O was prepared in a 10 kL culturing vessel, and the initial pH was adjusted to 6.0. After seeding 30 L of the culture solution, submerged culturing was conducted for 8 days under conditions of 26°C temperature, 360 m³/h aeration and 200 kPa interior pressure. The spinning was adjusted to maintain a dissolved oxygen concentration of 10-15 ppm. Also, the glucose concentration of the medium was adjusted by feeding to be in the range of 1-2.5% up to the 4th day, and 0.5-1% thereafter (where the percentages are based on weight (W/V)). After completion of the culturing, followed by filtering and drying, the cells containing triglycerides with arachidonic acid as a constituent fatty acid were collected, the oil/fat was obtained from the obtained cells by hexane extraction, and the edible oil/fat was subjected to a purification step (degumming, deacidification, steam distilation, decoloration) to obtain 150 kg of arachidonic acid-containing triglycerides (with arachidonic acid bound at any positions of the triglycerides). The obtained oil/fat (triglyceride) was methylesterified and the resulting fatty acid methyl ester was analyzed by gas chromatography, which revealed an arachidonic acid proportion of 40.84% of the total fatty acids. The palmitic acid, stearic acid, oleic acid, linoleic acid, γ-linolenic acid and dihomo-γ-linolenic acid contents were 11.63%, 7.45%, 7.73%, 9.14%, 2.23% and 3.27%, respectively. The arachidonic acid-containing oil/fat (triglyceride) was ethylesterified and 99% arachidonic acid ethyl ester was separated and purified from the fatty acid ethyl ester mixture comprising 40% arachidonic acid ethyl ester, by ordinary high performance liquid chromatography.

### Example 2

### Production of triglycerides including at least 5% of triglycerides with medium-chain fatty acids bound at the 1,3-positions and arachidonic acid bound at the 2-position (8A8)

After suspending 100 g of an ion-exchange resin support (Dowex MARATHON WBA^{™} : Dow Chemical) in 80 ml of 12.5% aqueous *Rhizopus delemarlipase* solution (Talipase powder, product of Tanabe Seiyaku), the suspension was dried under reduced pressure to obtain the immobilized lipase.

Next, 80 g of the triglyceride containing 40 wt% arachidonic acid obtained in Example 1 (TGA40S), 160 g of caprylic acid, 12 g of the aforementioned immobilized lipase and 4.8 ml of water were reacted at 30°C for 48 hours while stirring (130 rpm). After completion of the reaction, the reaction mixture was removed to obtain the activated immobilized lipase.

A 10 g portion of the immobilized enzyme *(Rhizopus delemar* lipase, support: Dowex MARATHON WBA^{™}) was packed into a jacketed glass column (1.8 x 12.5 cm, 31.8 ml volume), and a mixed oil containing the TGA40S obtained in Example 1 and caprylic acid in a proportion of 1:2 was passed through the column at a fixed flow rate (4 ml/h) for continuous reaction to obtain 400 g of reacted oil/fat. The column temperature was 40-41°C. The unreacted caprylic acid and free fatty acids were removed from the reacted oil/fat by molecular distillation, and the edible oil/fat was subjected to a purification step (degumming, deacidification, steam distillation, decoloration) to obtain 8A8-containing oil/fat (triglycerides). The 8A8 content of the obtained 8A8-containing oil/fat (triglycerides) was 31.6% as determined by gas chromatography and high performance liquid chromatography. (The proportions of 8P8, 808, 8L8, 8G8 and 8D8 were 0.6, 7.9, 15.1, 5.2 and 4.8%, respectively. The fatty acids P, O, L, G and D bound at the triglyceride 2-position represent palmitic acid, oleic acid, linoleic acid, γ-linolenic acid and dihomo-γ-linolenic acid, respectively, 8P8 is 1,3-capryloyl-2-palmitoleyl-glycerol, 808 is 1,3-capryloyl-2-oleoyl-glycerol, 8L8 is 1,3-capryloyl-2-linoleoyl-glycerol, 8G8 is 1,3-capryloyl-2-γ-linolenoyl-glycerol and 8D8 is 1,3-capryloyl-2-dihomo-γ-linolenoyl-glycerol.) Separation and purification by high performance liquid chromatography according to an established protocol yielded 96 mole percent 8A8 from the obtained 8A8-containing oil/fat (triglycerides).

### Example 3

### Production of test capsules

Water was added to 100 parts by weight of gelatin and 35 parts by weight of food grade glycerin for dissolution at 50-60°C to prepare a gelatin coating with a viscosity of 2000 cp. Next, 0.05 wt% vitamin E oil was mixed with the arachidonic acid-containing oil/fat (triglycerides) obtained in Example 1 to prepare Filling Content 1. Separately, 0.05 wt% vitamin E oil was mixed with oil/fat (triglycerides) containing 32 mole percent of the 8A8 obtained in Example 2 to prepare Filling Content 2. Filling Content 1 was used for molding and drying of capsules by a common method to manufacture soft capsules containing 200 mg of filling per capsule (arachidonic acid-containing edible oil capsules) while Filling Content 2 was also used for molding and drying of capsules by a common method to manufacture soft capsules containing 200 mg of filling per capsule (8A8-containing edible oil capsules). Soft capsules containing olive oil as the filling content were also manufactured as placebo capsules for a human test.

### Example 4

### Arachidonic acid-containing edible oil capsule ingestion test to determine effects on cognitive responses in healthy individuals

The event related potentials of brain (ERP) was measured by audible (or auditory discrimination) tasks according to the evoked potential measuring guidelines established by the Evoked Potential Examination Standards Committee of the Japan Society of Clinical Neurophysiology. Specifically, an auditory oddball paradigm was utilized for the ERP measurement and both ears of the test subject were stimulated by two different pure tones at frequencies of 1000 Hz and 2000 Hz were through headphones at ratio of 1:4 and in random order, and the subject was instructed to press a provided button when the 2000 Hz tone was heard and to count the number of times heard. The intensity of the tone was 90 dB, the duration was 100 msec and the inter stimulus interval was random between 1000-3000 msec. The approximate number of stimulations in the actual test was 200 per trial and the duration of the test was about 10 minutes. The test was conducted in 2 trials and the event related potentialss of brain were measured. Electroencephalographies (EEGs) were recorded by using Ag-AgCl electrodes, of which resistance is no greater than 5 kΩ, placed at three points along the mid-line of the scalp (Fz, Cz and Pz, according as international 10-20 displacements), and reference electrodes on both earlobes.

The maximum positive potential between 250-600 msec from the start of the low-frequency auditory simulation to be perceived (a 2000 Hz tone in this experiment) was identified as the intrinsic component P300 which varies in evaluation of selective attention or cognitive response, the time from the start of stimulation was recorded as the P300 latency (stimulation transmission speed) and the height of the potential from the baseline was recorded as the P300 amplitude (the amount of information processing resources).

The human test for the invention was conducted with due consideration to adhering to the spirit of the Helsinki Declaration.

Upon explanation for consent to test participation, 12 consenting healthy individuals (taking no medication, with no abnormal blood test results and with no infarctions as determined by cranial CT scan) were divided into two groups A and B (A: n=7, B: n=5). During a one month period, Group A was administered three of the arachidonic acid-containing edible oil capsules prepared in Example 3 (80 mg/capsule arachidonic acid) for a daily arachidonic acid intake of 240 mg, while Group B was administered three placebo capsules. The event related potentialss of brain were measured before and after capsule ingestion, and the P300 latency and amplitude were analyzed. The participants in Groups A and B were then taken off the capsules for a washout period of one month. After the washout period, Group A was administered the placebo capsules and Group B was administered the arachidonic acid-containing edible oil capsules for one month, and the event related potentialss of brain were measured in the same manner before and after capsule ingestion (double blind, crossover test).

Blood was taken at the time of event related potentials of brain measurement, and the total lipids were extracted from the serum of each participant by the Folch method. The lipids were fractionated by thin-layer chromatography, the phospholipid fraction was collected, the water was removed by azeotropic distillation with ethanol, and analysis was performed by gas chromatography upon conversion to fatty acid methyl ester with 10% HCl-methanol, to determine the arachidonic acid content of the serum phospholipids.

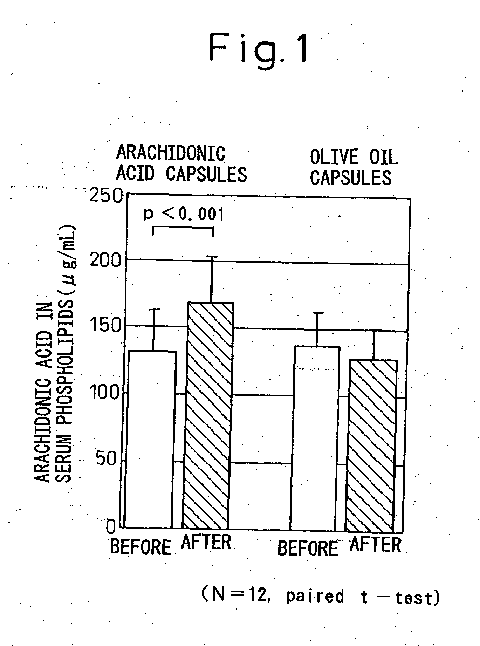

Fig. 1 shows changes in arachidonic acid contents in serum phospholipids before and after capsule ingestion. The arachidonic acid contents of the serum phospholipids of subjects ingesting the arachidonic acid-containing edible oil capsules were significantly increased after ingestion of the arachidonic acid-containing edible oil capsules, whereas the arachidonic acid contents of the serum phospholipids of subjects ingesting the placebo capsules were unchanged before and after ingestion of the placebo capsules.

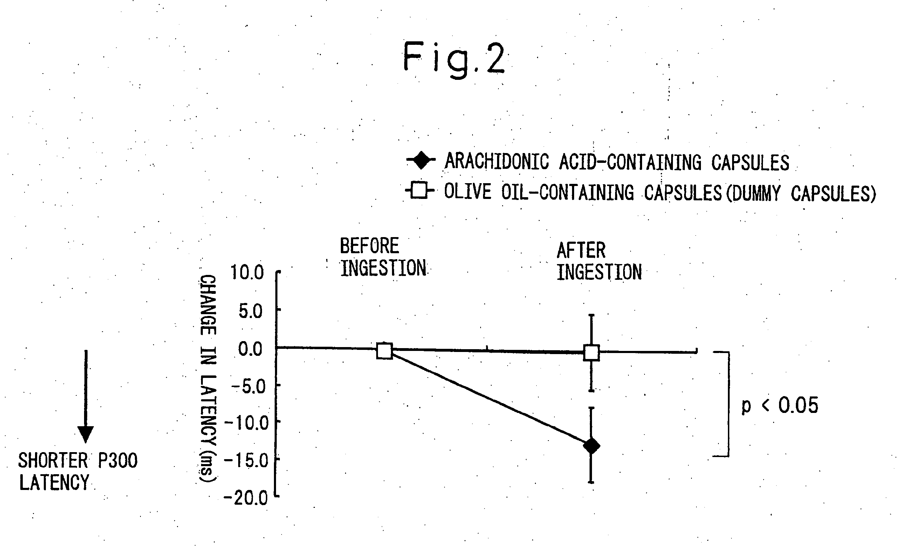

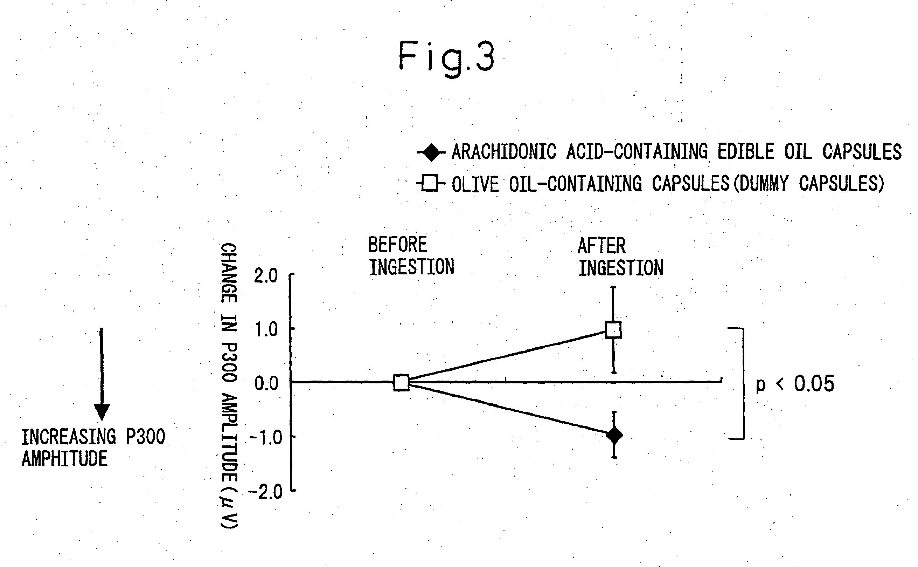

Fig. 2 and Fig. 3 show changes in P300 latency and amplitude before and after capsule ingestion. The P300 latency was significantly shortened by 12.3 msec and the P300 amplitude was significantly increased by 1.9 µV with ingestion of the arachidonic acid-containing edible oil capsules as compared to the placebo capsules. A normal shortening in P300 latency of 1.8 msec/year and decrease in P300 amplitude of 0.2 µV/year are known (Goodin DS et al. 1978), and therefore the results of this test indicate an average rejuvenation for cognitive responses of the subjects of 6.8 years based on P300 latency and 9.5 years based on P300 amplitude.

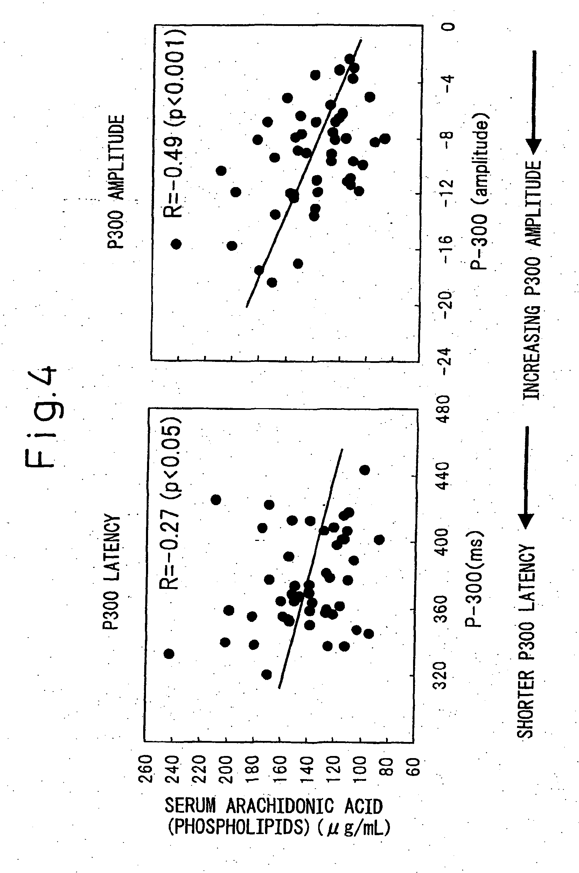

Next, the correlation between P300 (P300 latency and P300 amplitude) and serum arachidonic acid level (phospholipid) was determined by a first-order curve based on the least square method using total of 48 data obtained by measuring each of 12 subjects 4 times (Fig. 4). For P300 latency, a significant correlation (coefficient of correlation R = -0.27) was found between the arachidonic acid levels, with shorter latencies resulting from increased arachidonic acid levels. For P300 amplitude as well, a significant correlation (coefficient of correlation R = -0.49) was found between the arachidonic acid levels, with larger P300 amplitudes resulting from increased arachidonic acid levels. This constitutes the first demonstration of improvement of cognitive response by ingestion of arachidonic acid-containing edible oil, and the first proof of arachidonic acid as the cause of the effect.

### Example 5

### 8A8-containing edible oil capsule ingestion test to determine effects on cognitive responses in healthy individuals

Upon explanation for consent to test participation in the same manner as Example 4, 16 consenting healthy individuals (taking no medication, with no abnormal blood test results and with no infarctions as determined by cranial CT scan) were divided into two groups A and B (n=8 for each group). During a one month period, Group A was administered three of the 8A8-containing edible oil capsules prepared in Example 3 (72 mg/capsule arachidonic acid) while Group B was administered three placebo capsules, and upon measuring the event related potentialss of brain and recording the latency and amplitude before and after capsule ingestion (double-blind test), the P300 latency was found to be significantly shortened by 16.3 msec and the P300 amplitude significantly increased by 2.4 µV due to ingestion of the 8A8-containing edible oil capsules. These results indicate an average rejuvenation for cognitive responses of the subjects of 9.1 years based on P300 latency and 12.0 years based on P300 amplitude.

### Example 6

### Preparation of capsules containing oil/fat (triglycerides) with arachidonic acid as a constituent fatty acid

Water was added to 100 parts by weight of gelatin and 35 parts by weight of food grade glycerin for dissolution at 50-60°C to prepare a gelatin coating with a viscosity of 2000 cp. Next, 50 wt% of the arachidonic acid-containing oil/fat (triglycerides) obtained in Example 1 was mixed with 50 wt% fish oil (tuna oil comprising eicosapentaenoic acid and docosahexaenoic acid in proportions of 5.1% and 26.5%, respectively, of the total fatty acids), and this was combined with 0.05 wt% vitamin E oil to prepare Filling Content 3. Separately, 80 wt% of the arachidonic acid-containing oil/fat (triglycerides) was mixed with 20 wt% fish oil (tuna oil comprising eicosapentaenoic acid and docosahexaenoic acid in proportions of 5.1% and 26.5%, respectively, of the total fatty acids), and this was combined with 0.05 wt% vitamin E oil to prepare Filling Content 4. The 99% arachidonic acid ethyl ester obtained in Example 1 was also combined with 0.05 wt% vitamin E oil to obtain Filling Content 5. Filling Contents 3-5 were used for molding and drying of capsules by a common method to manufacture soft capsules containing 200 mg of filling per capsule.

### Example 7

### Use in oil infusion solutions

After combining 400 g of the oil/fat (triglycerides) containing 96% 8A8 obtained in Example 2, 48 g of purified egg yolk lecithin, 20 g of oleic acid, 100 g of glycerin and 40 ml of 0.1 N caustic soda and dispersing the mixture with a homogenizer, distilled water for injection was added to 4 liters. This was emulsified with a high-pressure spray-type emulsifier to prepare a lipid emulsion. The lipid emulsion was dispensed into plastic bags in portions of 200 ml and subjected to high-pressure steam sterilization treatment at 121°C for 20 minutes to prepare oil infusion solutions.

### Example 8 Use in juice

A 2 g portion of β-cyclodextrin was added to 20 ml of a 20% aqueous ethanol solution, and then 100 mg of the arachidonic acid-containing triglycerides obtained in Example 1 (comprising 0.05% vitamin E) was added thereto while stirring with a stirrer, and the mixture was incubated at 50°C for 2 hours. After cooling to room temperature (approximately 1 hour), incubation was continued at 4°C for 10 hours under continuous stirring. The produced precipitate was recovered by centrifugal separation, and after washing with n-hexane, it was lyophilized to obtain 1.8 g of a cyclodextrin clathrated compound containing arachidonic acid-containing triglycerides. A 1 g portion of this powder was uniformly mixed with 10 L of juice to prepare juice containing arachidonic acid-containing triglycerides.

## Claims

1. Use of a composition containing arachidonic acid and/or a compound in which arachidonic acid is a constituent fatty acid, for the treatment of a healthy adult human to improve or enhance normal responses of cognitive abilities so as to shorten P300 latency or augment P300 amplitude of the event related potentials of the brain (P300) as a response index of cognitive ability.

2. Use according to claim 1, wherein the compound with arachidonic acid as a constituent fatty acid is an arachidonic acid alcohol ester or a triglyceride, phospholipid or glycolipid containing arachidonic acid as part or all of the constituent fatty acid.

3. Use according to claim 2, wherein the triglyceride containing arachidonic acid as part or all of the constituent fatty acid is a triglyceride having medium-chain fatty acids bound at the 1,3-positions and arachidonic acid bound at the 2-position.

4. Use according to claim 3, wherein the medium-chain fatty acids are selected from fatty acids of 6 to 12 carbons.

5. Use according to claim 4, wherein the medium-chain fatty acids are selected from fatty acids of 8 carbons.

6. Use of a composition which contains triglycerides, including triglycerides containing arachidonic acid as part or all of the constituent fatty acid(s), for the treatment of a healthy adult human to improve or enhance normal responses of cognitive abilities so as to shorten P300 latency or augment P300 amplitude of the event related potentials of the brain (P300) as a response index of cognitive ability.

7. Use according to claim 6, in which the arachidonic acid content of the triglycerides including triglycerides containing arachidonic acid as part or all of the constituent fatty acid is at least 10 wt% of the total fatty acid in the triglycerides.

8. Use according to claim 6 or 7, wherein the triglycerides including triglycerides containing arachidonic acid as part or all of the constituent fatty acid are from microbes belonging to the genus *Mortierella.*

9. Use according to any one of claims 6 to 8, wherein the triglycerides including triglycerides containing arachidonic acid as part or all of the constituent fatty acid are triglycerides containing no eicosapentaenoic acid or containing not more than 1% eicosapentaenoic acid.

10. Use of a composition which contains triglycerides including at least 5 mole percent of triglycerides with medium-chain fatty acids bound at the 1,3-positions and arachidonic acid bound at the 2-position, for the treatment of a healthy adult human to improve or enhance normal responses cf cognitive abilities so as to shorten P300 latency or augment P300 amplitude of the event related potentials of the brain (P300) as a response index of cognitive ability.

11. Use according to claim 10, wherein the medium-chain fatty acids are selected from fatty acids of 6 to 12 carbons.

12. Use according to claim 11, wherein the medium-chain fatty acids are selected from fatty acids of 8 carbons.

13. Use according to any one of claims 1 to 12 in which said effects of improvement or enhancement of the normal responses of a healthy adult human are with respect to processing speed or response speed in relation to events selected from auditory stimuli, visual stimuli, olfactory stimuli, gustatory stimuli and somatosensory stimuli, as a said cognitive ability.

14. Use according to any one of claims 1 to 12 in which said effects of improvement or enhancement of normal responses of a healthy adult human are with respect to the concentration ability in relation to events selected from auditory stimuli, visual stimuli, olfactory stimuli, gustatory stimuli and somatosensory stimuli, as a said cogniLive ability.

15. Use according to any one of claims 1 to 12 in which said effects of improvement or enhancement of normal responses are with respect to the awareness level of a healthy adult human, as said cognitive ability.

16. Use according to any one of claims 1 to 12 in which said effects of improvement or enhancement of normal responses are with respect to discriminatory ability of a healthy adult human in relation to events selected from auditory stimuli, visual stimuli, olfactory stimuli, gustatory stimuli and somatosensory stimuli, as a said cognitive ability.

17. Use according to any one of the preceding claims in which the composition is a food composition or pharmaceutical composition.

18. Use according to claim 17 in which the composition is a food composition containing the arachidonic acid and/or compound in which arachidonic acid is a constituent fatty acid, in an amount such that the daily ingestion for an adult is 0.001-20 g in terms of arachidonic acid.

19. Use according to claim 17 cr 18 in which the composition is a food composition which contains at least 0.001 wt% of triglycerides having medium-chain fatty acids bound at the 1,3-positions and arachidonic acid bound at the 2-position.

20. Use according to any one of claims 17 to 19 in which the composition is a functional food, nutritional supplement food, special health care food or geriatric food.

21. Use according to any one of the preceding claims in which the composiLion further comprises docosahexaenoic acid and/or compound in which docosahexaenoic acid is a constituent fatty acid.

22. Use according to claim 21 wherein the compound in which docosahexaenoic acid is a constituent fatty acid is a docosahexaenoic acid alcohol ester or a triglyceride, phospholipid or glycolipid comprising docosahexaenoic acid as part or all of the constituent fatty acid(s).

23. Use according to claim 21 cr 22 in which the arachidonic acid/docosahexaenoic acid ratio (by weight) in the combination of the arachidonic acid and docosahexaenoic acid is in the range of 0.1-15.

24. Use according to any one of claims 1 to 23 in which eicosapentaenoic acid is also present in the composition in an amount not exceeding 1/5 of the arachidonic acid in the composition.

## Patentansprüche

1. Verwendung einer Zusammensetzung, die Arachidonsäure und/oder eine Verbindung enthält, in der Arachidonsäure ein Fettsäurebestandteil ist, zur Behandlung eines gesunden erwachsenen Menschen zur Verbesserung oder Steigerung von Normalreaktionen von kognitiven Fähigkeiten, um bezogen auf das ereignisbezogene Potenzial des Gehirns (P300) als Reaktionsindex der kognitiven Fähigkeit die P300-Latenz zu verkürzen oder die P300-Amplitude zu erhöhen.

2. Verwendung nach Anspruch 1, wobei die Verbindung mit Arachidonsäure als ein Fettsäurebestandteil ein Arachidonsäurealkoholester oder ein Triglycerid, Phospholipid oder Glycolipid ist, das Arachidonsäure als Teil oder Ganzes des Fettsäurebestandteils enthält.

3. Verwendung nach Anspruch 2, wobei das Triglycerid, das als Teil oder Ganzes des Fettsäurebestandteils Arachidonsäure enthält, ein Triglycerid mit mittelkettigen Fettsäuren, die an den 1,3-Positionen gebunden sind, und Arachidonsäure ist, die an der 2-Position gebunden ist.

4. Verwendung nach Anspruch 3, wobei die mittelkettigen Fettsäuren ausgewählt sind aus Fettsäuren mit 6 bis 12 Kohlenstoffen.

5. Verwendung nach Anspruch 4, wobei die mittelkettigen Fettsäuren ausgewählt sind aus Fettsäuren mit 8 Kohlenstoffen.

6. Verwendung einer Zusammensetzung, die Triglyceride enthält, einschließlich Triglyceride, die Arachidonsäure als Teil oder Ganzes des Fettsäure(n)bestandteils enthalten, zur Behandlung eines gesunden erwachsenen Menschen zur Verbesserung oder Steigerung von Normalreaktionen von kognitiven Fähigkeiten, um bezogen auf das ereignisbezogene Potenzial des Gehirns (P300) als Reaktionsindex der kognitiven Fähigkeit die P300-Latenz zu verkürzen oder die P300-Amplitude zu erhöhen.

7. Verwendung nach Anspruch 6, in der der Arachidonsäuregehalt der Triglyceride, einschließlich der Triglyceride, die Arachidonsäure als Teil oder Ganzes des Fettsäurebestandteils enthalten, mindestens 10 Gew.-% der gesamten Fettsäure der Triglyceride ist.

8. Verwendung nach Anspruch 6 oder 7, wobei die Triglyceride, einschließlich der Triglyceride, die Arachidonsäure als Teil oder Ganzes des Fettsäurebestandteils enthalten, von Mikroben sind, die dem Genus Mortierella angehören.

9. Verwendung nach einem der Ansprüche 6 bis 8, wobei die Triglyceride, einschließlich der Triglyceride, die Arachidonsäure als Teil oder Ganzes des Fettsäurebestandteils enthalten, Triglyceride sind, die keine Eicosapentaensäure oder nicht mehr als 1 % Eicosapentaensäure enthalten.

10. Verwendung einer Zusammensetzung, die Triglyceride enthält, die mindestens 5 Mol-% Triglyceride mit mittelkettigen Fettsäuren, die an den 1,3-Positionen gebunden sind, und Arachidonsäure, die an der 2-Position gebunden ist, umfassen, zur Behandlung eines gesunden erwachsenen Menschen zur Verbesserung oder Steigerung von Normalreaktionen von kognitiven Fähigkeiten, um bezogen auf das ereignisbezogene Potenzial des Gehirns (P300) als Reaktionsindex der kognitiven Fähigkeit die P300-Latenz zu verkürzen oder die P300-Amplitude zu erhöhen.

11. Verwendung nach Anspruch 10, wobei die mittelkettigen Fettsäuren ausgewählt sind aus Fettsäuren mit 6 bis 12 Kohlenstoffen.

12. Verwendung nach Anspruch 11, wobei die mittelkettigen Fettsäuren ausgewählt sind aus Fettsäuren mit 8 Kohlenstoffen.

13. Verwendung nach einem der Ansprüche 1 bis 12, in der die Effekte der Verbesserung oder Steigerung der Normalreaktionen eines gesunden erwachsenen Menschen, was Ablaufgeschwindigkeit oder Reaktionsgeschwindigkeit betrifft, bezogen sind auf Ereignisse ausgewählt aus auditiven Reizen, visuellen Reizen, Geruchsreizen, Geschmacksreizen und somatosensorischen Reizen als eine besagte kognitive Fähigkeit.

14. Verwendung nach einem der Ansprüche 1 bis 12, in der die Effekte der Verbesserung oder Steigerung der Normalreaktionen eines gesunden erwachsenen Menschen, was die Konzentrationsfähigkeit betrifft, bezogen sind auf Ereignisse, ausgewählt aus auditiven Reizen, visuellen Reizen, Geruchsreizen, Geschmacksreizen und somatosensorischen Reizen als eine besagte kognitive Fähigkeit.

15. Verwendung nach einem der Ansprüche 1 bis 12, in der die Effekte der Verbesserung oder Steigerung der Normalreaktionen auf den Bekanntheitsgrad für einen gesunden erwachsenen Menschen als eine besagte kognitive Fähigkeit bezogen sind.

16. Verwendung nach einem der Ansprüche 1 bis 12, in der die Effekte der Verbesserung oder Steigerung der Normalreaktionen, was Unterscheidungsfähigkeit eines gesunden erwachsenen Menschen betrifft, bezogen sind auf Ereignisse ausgewählt aus auditiven Reizen, visuellen Reizen, Geruchsreizen, Geschmacksreizen und somatosensorischen Reizen als eine besagte kognitive Fähigkeit.

17. Verwendung nach einem der vorangegangenen Ansprüche, wobei die Zusammensetzung eine Lebensmittelzusammensetzung oder eine pharmazeutische Zusammensetzung ist.

18. Verwendung nach Anspruch 17, wobei die Zusammensetzung eine Lebensmittelzusammensetzung ist, die Arachidonsäure und/oder eine Verbindung enthält, in der Arachidonsäure ein Fettsäurebestandteil ist, in einer solchen Menge, dass die tägliche Aufnahme für einen Erwachsenen 0,001-20 g bezogen auf Arachidonsäure ist.

19. Verwendung nach Anspruch 17 oder 18, wobei die Zusammensetzung eine Lebensmittelzusammensetzung ist, die mindestens 0,001 Gew.-% Triglyceride mit mittelkettigen Fettsäuren, die an den 1,3-Positionen gebunden sind, und Arachidonsäure, die an der 2-Position gebunden ist, enthält.

20. Verwendung nach einem der Ansprüche 17 bis 19, wobei die Zusammensetzung ein funktionelles Lebensmittel, Nahrungsergänzungsmittel, spezielle Gesundheitsnahrung oder ein geriatrisches Nahrungsmittel ist.

21. Verwendung nach einem der vorangegangenen Ansprüche, wobei die Zusammensetzung weiters Docosahexaensäure und/oder eine Verbindung umfasst, in der Docosahexaensäure ein Fettsäurebestandteil ist.

22. Verwendung nach Anspruch 21, wobei die Verbindung, in der Docosahexaensäure ein Fettsäurebestandteil ist, ein Docosahexaensäurealkoholester oder ein Triglycerid, Phospholipid oder Glycolipid, umfassend Docosahexaensäure als Teil oder Ganzes des Fettsäure(n)bestandteils, ist.

23. Verwendung nach Anspruch 21 oder 22, wobei das Verhältnis (bezogen auf Gew.) der Arachidonsäure/Docosahexaensäure in der Kombination der Arachidonsäure und Docosahexaensäure im Bereich von 0,1 - 15 ist.

24. Verwendung nach einem der Ansprüche 1 bis 23, wobei auch Eicosapentaensäure in der Zusammensetzung in einer Menge nicht höher als 1/5 der Arachidonsäure in der Zusammensetzung vorhanden ist.

## Revendications

1. Utilisation d'une composition contenant de l'acide arachidonique et/ou d'un composé dans lequel l'acide arachidonique est un acide gras constitutif, pour le traitement d'un humain adulte en bonne santé pour améliorer ou intensifier les réponses normales des capacités cognitives de manière à raccourcir la latence P300 ou à augmenter l'amplitude P300 des potentiels relatifs aux événements du cerveau (P300) en tant qu'indice de réponse de capacité cognitive.

2. Utilisation selon la revendication 1, dans laquelle le composé avec acide arachidonique comme acide gras constitutif est un ester d'alcool de l'acide arachidonique ou un triglycéride, un phospholipide ou un glycolipide contenant de l'acide arachidonique comme partie ou tout de l'acide gras constitutif.

3. Utilisation selon la revendication 2, dans laquelle le triglycéride contenant de l'acide arachidonique comme partie ou tout de l'acide gras constitutif est un triglycéride ayant des acides gras à chaîne moyenne liés sur les positions 1,3 et de l'acide arachidonique lié sur la position 2.

4. Utilisation selon la revendication 3, dans laquelle les acides gras à chaîne moyenne sont sélectionnés parmi les acides gras de 6 à 12 carbones.

5. Utilisation selon la revendication 4, dans laquelle les acides gras à chaîne moyenne sont sélectionnés parmi les acides gras de 8 carbones.

6. Utilisation d'une composition qui contient des triglycérides, y compris des triglycérides contenant l'acide arachidonique comme partie ou tout de/des acide(s) gras constitutif(s), pour le traitement d'un humain adulte en bonne santé pour améliorer ou intensifier les réponses normales des capacités cognitives de manière à raccourcir la latence P300 ou à augmenter l'amplitude P300 des potentiels relatifs aux événements du cerveau (P300) en tant qu'indice de réponse de capacité cognitive.

7. Utilisation selon la revendication 6, dans laquelle le contenu en acide arachidonique des triglycérides comprenant des triglycérides contenant l'acide arachidonique comme partie ou tout de l'acide gras constitutif est d'au moins 10 % en poids de l'acide gras total dans les triglycérides.

8. Utilisation selon la revendication 6 ou 7, dans laquelle les triglycérides comprenant des triglycérides contenant l'acide arachidonique comme partie ou tout de l'acide gras constitutif proviennent de microbes appartenant au genre *Mortierella.*

9. Utilisation selon l'une quelconque des revendications 6 à 8, dans laquelle les triglycérides comprenant des triglycérides contenant l'acide arachidonique comme partie ou tout de l'acide gras constitutif sont des triglycérides ne contenant aucun acide eicosapentaénoïque ou ne contenant pas plus de 1 % d'acide eicosapentaénoïque.

10. Utilisation d'une composition qui contient des triglycérides comprenant au moins 5 pourcent en mole de triglycérides avec des acides gras à chaîne moyenne liés sur les positions 1,3 et de l'acide arachidonique lié sur la position 2, pour le traitement d'un humain adulte en bonne santé pour améliorer ou intensifier des réponses normales des capacités cognitives de manière à raccourcir la latence P300 ou à augmenter l'amplitude P300 des potentiels relatifs aux événements du cerveau (P300) en tant qu'indice de réponse de capacité cognitive.

11. Utilisation selon la revendication 10, dans laquelle les acides gras à chaîne moyenne sont sélectionnés parmi les acides gras de 6 à 12 carbones.

12. Utilisation selon la revendication 11, dans laquelle les acides gras à chaîne moyenne sont sélectionnés parmi les acides gras de 8 carbones.

13. Utilisation selon l'une quelconque des revendications 1 à 12, dans laquelle lesdits effets d'amélioration ou d'intensification des réponses normales d'un humain adulte en bonne santé sont en rapport avec la vitesse de traitement ou la vitesse de réponse en relation avec des événements sélectionnés parmi les stimuli auditifs, les stimuli visuels, les stimuli olfactifs, les stimuli gustatifs et les stimuli somato-sensoriels, en tant que ladite capacité cognitive.

14. Utilisation selon l'une quelconque des revendications 1 à 12, dans laquelle lesdits effets d'amélioration ou d'intensification des réponses normales d'un humain adulte en bonne santé sont en rapport avec la capacité de concentration en relation avec des événements sélectionnés parmi les stimuli auditifs, les stimuli visuels, les stimuli olfactifs, les stimuli gustatifs et les stimuli somato-sensoriels, en tant que ladite capacité cognitive.

15. Utilisation selon l'une quelconque des revendications 1 à 12, dans laquelle lesdits effets d'amélioration ou d'intensification des réponses normales sont en relation avec le niveau de conscience d'un humain adulte en bonne santé, en tant que ladite capacité cognitive.

16. Utilisation selon l'une quelconque des revendications 1 à 12, dans laquelle lesdits effets d'amélioration ou d'intensification des réponses normales sont en rapport avec la capacité discriminatoire d'un humain adulte en bonne santé en relation avec des événements sélectionnés parmi les stimuli auditifs, les stimuli visuels, les stimuli olfactifs, les stimuli gustatifs et les stimuli somato-sensoriels, en tant que ladite capacité cognitive.

17. Utilisation selon l'une quelconque des revendications précédentes dans laquelle la composition est une composition alimentaire ou une composition pharmaceutique.

18. Utilisation selon la revendication 17, dans laquelle la composition est une composition alimentaire contenant l'acide arachidonique et/ou un composé dans lequel l'acide arachidonique est un acide gras constitutif, dans une quantité telle que l'ingestion quotidienne pour un adulte est de 0,001-20 g en termes d'acide arachidonique.

19. Utilisation selon la revendication 17 ou 18, dans laquelle la composition est une composition alimentaire qui contient au moins 0,001% en poids de triglycérides ayant des acides gras à chaîne moyenne liés sur les positions 1,3 et de l'acide arachidonique lié sur la position 2.

20. Utilisation selon l'une quelconque des revendications 17 à 19, dans laquelle la composition est un aliment fonctionnel, un aliment en complément nutritionnel, un aliment spécial soins de santé ou un aliment gériatrique.

21. Utilisation selon l'une quelconque des revendications précédentes dans laquelle la composition comprend en outre de l'acide docosahexaénoïque et/ou un composé dans lequel l'acide docosahexaénoïque est un acide gras constitutif.

22. Utilisation selon la revendication 21, dans laquelle le composé où l'acide docosahexaénoïque est un acide gras constitutif est un ester d'alcool de l'acide docosahexaénoïque ou un triglycéride, un phospholipide ou un glycolipide comprenant l'acide docosahexaénoïque comme partie ou tout du/des acide(s) gras constitutif(s).

23. Utilisation selon la revendication 21 ou 22, dans laquelle le rapport acide arachidonique/acide docosahexaénoïque (en poids) dans la combinaison de l'acide arachidonique et acide docosahexaénoïque se situe dans l'intervalle entre 0,1 et 15.

24. Utilisation selon l'une quelconque des revendications 1 à 23, dans laquelle l'acide eicosapentaénoïque est également présent dans la composition dans une quantité ne dépassant pas 1/5 de l'acide arachidonique dans la composition.
